(19) Europäisches Patentamt
European Patent Office
Office européen des brevets

(11) Veröffentlichungsnummer : **0 024 517**
**B1**

(12) **EUROPÄISCHE PATENTSCHRIFT**

(45) Veröffentlichungstag der Patentschrift : **09.06.82**

(51) Int. Cl.³ : **C 07 C 47/02**, C 07 C 45/29, C 07 C 45/41, A 61 K 7/46, C 11 B 9/00

(21) Anmeldenummer : **80103923.1**

(22) Anmeldetag : **09.07.80**

# (54) 3-Methyl-aldehyde, Verfahren zu ihrer Herstellung und ihre Verwendung als Riechstoffe.

(30) Priorität : **20.07.79 DE 2929340**

(43) Veröffentlichungstag der Anmeldung : **11.03.81 (Patentblatt 81/10)**

(45) Bekanntmachung des Hinweises auf die Patenterteilung : **09.06.82 Patentblatt 82/23**

(84) Benannte Vertragsstaaten : **CH DE FR GB IT LI NL**

(56) Entgegenhaltungen :
**FR A1 2 361 325**

**US A 3 959 396**

**TETRAHEDRON LETTERS, Band 1972, Nr. 13, Oxford-New York-Braunschweig K. SAKAI et al. « Synthetic Studies on Prostanoids. Synthesis of Methyl-9-Oxoprostanoate », Seiten 1287 bis 1290**

**Chemical Abstracts Band 83, Nr. 5, 1975 Columbus, Ohio, USA A. MATSUDA « Hydroformylation and reductive esterification catalysts for olefins », Seite 458, Spalte 1, Abstract Nr. 42853z**

(73) Patentinhaber : **HAARMANN & REIMER GMBH**
**Postfach 138**
**D-3450 Holzminden (DE)**

(72) Erfinder : **Bauer, Kurt, Dr.**
**Corveyblick 41**
**D-3450 Holzminden (DE)**
Erfinder : **Hagena, Detlef, Dr.**
**Kleiner Bruch 3**
**D-3470 Höxter (DE)**
Erfinder : **Müller, Hans-Otto, Dr.**
**Jasper-Strasse 32**
**D-3450 Holzminden (DE)**

(74) Vertreter : **Dill, Erwin, Dr. et al**
**c/o BAYER AG Zentralbereich Patente Marken und Lizenzen Bayerwerk**
**D-5090 Leverkusen 1 (DE)**

**EP 0 024 517 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 18, rue St-Denis, 75001 Paris, France

# 3-Methyl-aldehyde, Verfahren zu ihrer Herstellung und ihre Verwendung als Riechstoffe

Die Erfindung betrifft 3-Methylaldehyde der Formel

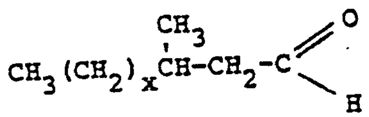 (I)

in der

x für 6 oder 7 steht.

Die Erfindung betrifft ferner Verfahren zur Herstellung der 3-Methylaldehyde der Formel (I). Die Verfahren sind dadurch gekennzeichnet, daß man entweder eine Säure der Formel

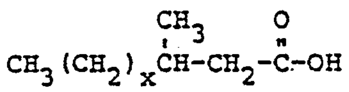 (II)

in der

x die unter Formel I angegebene Bedeutung hat

nach für die Reduktion von Carbonsäuren zu Aldehyden bekannten Verfahren, reduziert, oder einen Alkohol der Formel

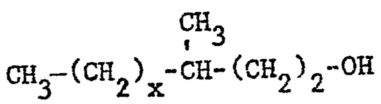 (III)

in der

x die unter Formel I angegebene Bedeutung hat,

nach für die Oxydation von Alkoholen zu Aldehyden bekannten Verfahren oxydiert.

Außerdem betrifft die Erfindung die Verwendung der 3-Methylaldehyde der Formel (I) als Riechstoffe.

Die Reduktion der Säuren kann mit Ameisensäure an einem Mangan-(II)-oxid-Katalysator in der Gasphase erfolgen. Dazu werden die Säure und ein 2- bis 6-facher, vorzugsweise 4- bis 5-facher molarer überschuß an Ameisensäure getrennt unter Vakuum verdampft und bei einer Reaktionstemperatur von 340 bis 400 °C, vorzugsweise 360 °C, über einen Mangan-(II)-oxid-Bimsstein-Katalysator geleitet.

Eine weitere Möglichkeit ist die Reduktion nach Rosenmund. Dazu werden die Säuren mit Thionylchlorid, Phosphortrichlorid oder Phosphorpentachlorid in das Säurechlorid überführt und dieses mit Wasserstoff und einem inaktivierten Palladium-Katalysator zum Aldehyd reduziert. Der Palladium-Katalysator, 5 % Palladium auf einem Träger wie Kieselgur, Calciumcarbonat, Aktivkohle, vorzugsweise aber Bariumsulfat, wird in einer Menge von 5 bis 10 Gew.-%, bezogen auf das Säurechlorid verwendet. Die Inaktivierung erfolgt durch einen Zusatz von Chinolin-Schwefel, Thioharnstoff oder Phenylsenföl. Bei der Verwendung von Thionylchlorid zur Herstellung des Säurechlorides kann die Inaktivierung entfallen, da die im Säurechlorid verbleibenden Verunreinigungen ausreichen, den Katalysator zu desaktivieren. Die Reduktion wird in einem inerten Lösungsmittel wie Xylol, Toluol oder Tetralin bei Temperaturen zwischen 100 und 180 °C durchgeführt.

Die Oxidation der Alkohole der Formel (III) zu den Aldehyden der Formel (I) kann mit Chromsäure in Schwefelsäure vorgenommen werden. Als vorteilhaft hat sich jedoch die katalytische Dehydrierung erwiesen, bei der als Katalysator Kupfer-, Silber- oder Zinkverbindungen, vorzugsweise Kupfer-Chromoxid, eingesetzt werden. Die Reaktion kann als Sumpfphasendehydrierung beim Siedepunkt des Alkohols mit 10 Gew.-% Katalysator oder als Dampfphasendehydrierung bei 400 bis 700 °C an einem festen Kontakt durchgeführt werden.

Die Herstellung der Säuren der Formel (II) kann nach folgendem Verfahren erfolgen : Hexan- bzw. Heptansäurechlorid werden mit Dimethylacrylsäureethylester in Gegenwart eines Friedel-Crafts-Katalysators zu einem einfach ungesättigten 3-Methyl-5-oxo-alkansäure-ethyl-ester umgesetzt. Aus diesem Ester werden die Säuren der Formel (II) durch Hydrierung in Gegenwart von Raney-Nickel und nachfolgende Reduktion mit Hydrazin nach Wolff-Kishner hergestellt.

Die Alkohole der Formel (III) können aus den Säuren der Formel (II) durch Reduktion mit Lithiumaluminiumhydrid erhalten werden. Als vorteilhaft hat es sich erwiesen, die Säuren zunächst in die Ethylester zu überführen, um diese dann mit Lithiumaluminiumhydrid zu reduzieren oder im Autoklaven mit Wasserstoff und einem Kupfer-Chromoxid-Katalysator zu hydrieren.

Die erfindungsgemäß 3-Methylaldehyde sind wertvolle Riechstoffe, die eine citrusartig-wachsige Note aufweisen, als strahlend, kräftig, haftfest und harmonisch charakterisiert werden können und besonders zur Geruchskomplexbildung mit anderen Komponenten geeignet sind.

Darüber hinaus weist das 3-Methyldecanal eine cyclamenartigozonige Grünnote auf, wie man sie in der Schale südamerikanischer Limette-Früchte findet, während das 3-Methylundecanal sehr schön den Kopfgeruch der Mandarinenschale verkörpert.

Gegenüber den als Riechstoffen bekannten isomeren 2-Methyl-aldehyden zeichnen sich die erfindungsgemäßen 3-Methyl-adehyde durch neuartige Geruchsnoten und einen feineren, und in sich stärker abgerundeten Geruch aus.

Die erfindungsgemäßen Verbindungen werden in Mischungen mit anderen Riechstoffen in Riechstoffkompositionen, z.B. in Mengen von 0,01 bis 20 Gew.-%, vorzugsweise in Mengen von etwa 0,1 bis 10 Gew.-%, bezogen auf das Gesamtgewicht eingesetzt.

Das Anwendungsgebiet der erfindungsgemäßen Verbindungen ist wegen ihres harmonischen

Geruchstyps und ihrer vorteilhaften anwendungstechnischen Eigenschaften, z.B. der Stabilität gegenüber aggressiven Medien, ungewöhnlich groß. Sie eignen sich für die Verwendung in Parfümkompositionen für die verschiedensten Fertigungsprodukte z.B. für hochwertige Kosmetika, für Feinparfümerieartikel wie Extraits, Seifen, Deo-Sprays, Shampoos, Schaumbäder und für Detergentien.

Beispiel 1

546,6 g (3,17 Mol) 3-Methyl-decanol werden mit 54,6 g Kupfer-Chromoxid auf Siedetemperatur erhitzt. Unter Wasserstoff-Entwicklung gehen 428 g Destillat über. Daraus werden durch Feindestillation 156,2 g 3-Methyl-decanol zurückgewonnen. Es werden 244,4 g 3-Methyl-decanal mit einem Siedepunkt von 46 °C/0,1 Torr erhalten, entsprechend einer Ausbeute von 63,4 % der Theorie, bezogen auf umgesetzten Alkohol.

Das als Ausgangsmaterial verwendete 3-Methyldecanol wird nach folgendem Verfahren hergestellt :

700 g (5,2 Mol) Hexansäurechlorid und 666 g (5,2 Mol) Dimethylacrylsäureethylester werden gleichzeitig innerhalb von 3 Stunden bei 30 °C zu einer Suspension von 1 533 g (11,48 Mol) Aluminiumchlorid in 850 ml Methylenchlorid zudosiert. Nach beendeter Zugabe wird die Reaktionsmischung 3 Stunden auf Rückflußtemperatur erwärmt und dann mit Eis-Wasser hydrolysiert. Die wäßrige Phase wird zweimal mit je 150 ml Methylenchlorid extrahiert, die vereinigten organischen Phasen werden neutralgewaschen und das Lösungsmittel abgezogen. Man erhält 1 130 g rohen 3-Methyl-5-keto-decen-2(3)-säureethylester ($Kp_{0,3}$ : 93 °C), entsprechend einer Ausbeute von 96 % der Theorie.

1 130 g (5 Mol) 3-Methyl-5-keto-decen-2(3)-säureethylester werden mit 336 g (6 Mol) Kaliumhydroxid, 400 ml Ethanol und 1 300 ml Wasser in einen Autoklaven gegeben und unter Zusatz von 120 g Raney-Nickel bei 40 °C und 40 Atm Wasserstoffdruck hydriert. Die Wasserstoffaufnahme beträgt 87 % der Theorie. Nach Abfiltrieren des Katalysators wird die Reaktionslösung mit Schwefelsäure angesäuert und mit Essigester extrahiert. Die Essigesterlösung wird abgetrennt und vom Lösungsmittel befreit. Es verbleiben 915 g rohe 3-Methyl-5-keto-decansäure.

Diese rohe 3-Methyl-5-keto-decansäure wird ohne weitere Reinigung mit 473 ml Diethylenglykol, 1 260 g (22,5 Mol) Kaliumhydroxid und 850 g (17 Mol) 80 %igem Hydrazinhydrat 2 Stunden auf Rückflußtemperatur erhitzt. Dann wird Wasser und überschüssiges Hydrazin abdestilliert und die Reaktionstemperatur für 1 Stunde auf 210 bis 220 °C gesteigert. Nach dem Abkühlen wird die Reaktionsmischung mit 400 ml Toluol und 400 ml Wasser versetzt, mit Salzsäure angesäuert und auf eine Mischung aus Wasser und Eis gegossen. Die organische Phase wird abgetrennt ; die wäßrige Phase wird zweimal mit Toluol extrahiert. Die wereinigten organischen Phasen werden neutralgewaschen und von Lösungsmittel befreit. Es verbleiben 707,8 g 3-Methyldecansäure ($Kp_{0,8}$ : 110 °C), entsprechend einer Ausbeute von 76,1 % der Theorie, bezogen auf 3-Methyl-5-keto-decen-2(3)-säureethylester.

707,8 g (3,8 Mol) 3-Methyl-decansäure werden in 305,9 g Ethanol und 700 ml Toluol, das mit 19 g p-Toluolsulfonsäure versetzt ist, 6 Stunden unter Rückfluß am Wasserabscheider erhitzt. Nach der Aufarbeitung erhält man 740,5 g 3-Methyl-decansäureethylester ($Kp_{0,5}$ : 75 °C), entsprechend einer Ausbeute von 91 % der Theorie.

740 g (3,46 Mol) 3-Methyl-decansäureethylester, gelöst in 600 ml Ether, werden unter Kühlung zu einer Suspension von 72 g (1,89 Mol) Lithiumaluminiumhydrid in 600 ml Ether gegeben. Nach beendeter Zugabe wird die Reaktionsmischung eine Stunde auf Rückflußtemperatur erhitzt, dann hydrolysiert und mit Schwefelsäure angesäuert. Die organische Phase wird abgetrennt, die wäßrige Phase mit Ether extrahiert und die vereinigten organischen Phasen vom Lösungsmittel befreit. Die Destillation des Rückstandes ergibt 529,5 g 3-Methyldecanol ($Kp_{0,3}$ : 83 °C), entsprechend einer Ausbeute von 88,9 % der Theorie.

Beispiel 2

286,8 g (1,54 Mol) 3-Methyl-undecanol werden analog Beispiel 1 mit 28,7 g Kupfer-Chromoxid einer Sumpfphasendehydrierung unterworfen. Man erhält 126,6 g 3-Methyl-undecanal mit einem Siedepunkt von 56 °C/0,02 Torr, entsprechend einer Ausbeute von 64,5 % der Theorie, bezogen auf umgesetzten Alkohol.

Die Ausgangsverbindung 3-Methyl-undecanol ($Kp_{0,5}$ : 105 °C) wird analog der in Beispiel 1 beschriebenen Herstellung von 3-Methyldecanol aus Hexansäurechlorid und Dimethyl-acrylsäureethylester hergestellt.

Beispiel 3

Eine Parfümkomposition mit Citrusnote wird durch Mischen folgender Komponenten hergestellt :

| | |
|---|---|
| 200 | Citronenöl-Terpene |
| 30 | Citral |
| 20 | Citrathal PPL |
| 5 | Geranitril |
| 3 | Tagetesöl |
| 2 | Trimethyltetrahydrobenzaldehyd |
| 5 | Trimethylundecylenaldehyd |
| 50 | Petitgrainöl Paraguay |
| 100 | Menthanylacetat |
| 250 | Terpineol |
| 10 | Corianderöl |
| 5 | Rosmarinöl |
| 20 | Galbanum-Resinoid |
| 50 | Dihydro-nor-dicylopentadienylacetat |
| 20 | Styrolylacetat |
| 5 | Eugenol |
| 50 | α-Hexylzimtaldehyd |
| 5 | 2-n-Heptyl-cyclopentanon-1 |

10 p-tert.-Butyl-α-methyl-hydrozimtaldehyd
30 Benzylsalicylat
10 Pentadecanolid, 50 %ig in Triethylcitrat
70 Diethylphthalat
30 Phenoxiethylisobutyrat

100 Gewichtsteile

Durch Zugabe von 20 Gew.-Teilen 3-Methyldecanal erhält die Komposition eine lebhafte Frische, Fülle und Harmonie.

Beispiel 4

Eine Parfümkomposition mit exotischer Note wird durch Mischen der folgenden Komponenten hergestellt :

100 Bergamottöl Reggio
50 Orangenöl Florida
10 Nelkenblütenöl
40 Eugenol
30 Geranylacetat
5 Galbanum-Resinoid
3 Bayöl
2 Zimtrindenöl
1 Cistusöl
10 Perubalsamöl
10 Zimtalkohol ex Storax
70 β-Phenylethylalkohol
10 Rose abs. de Mai
5 Hyacinthe abs.
20 Ylang-Ylangöl
5 Jasmin abs.
10 Methyldihydrojasmonat
1 Undecalacton
50 Hydroxicitronellal
20 p-tert. Butyl-α-methylzimtaldehyd
5 Styrolylacetat
1 Allyljonon
30 γ-Methyljonon
10 Heliotropin
100 Benzylsalicylat
30 Sandelholzöl Mysore (ostindisch)
50 Acetylcedren
30 Patchouliöl Singapore
20 Vetiverylacetat
5 Eichenmoos abs. 50 %ig in Triethylcitrat
1 Zibeth
20 Cumarin
3 Ethylvanillin
20 Moschus Keton
213 Diethylphthalat

990 Gewichtsteile

Durch Zusatz von 10 Gew.-Teilen 3-Methylundecanal erhält die Komposition Eleganz, Strahlkraft und die charakteristische Frische, wie sie in exotischen Parfüms gewünscht wird.

**Ansprüche**

1. 3-Methyl-aldehyde der allgemeinen Formel

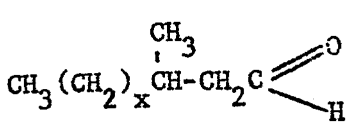

in der
x für 6 oder 7 steht.

2. Verfahren zur Herstellung von Verbindungen der allgemeinen Formel

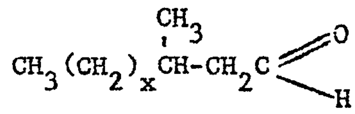

in der
x für 6 oder 7 steht,
dadurch gekennzeichnet, daß man
a) eine Carbonsäure der allgemeinen Formel

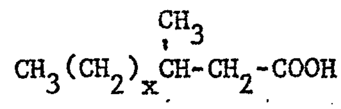

in der
x die vorstehend genannte Bedeutung hat, reduziert oder
b) einen Alkohol der allgemeinen Formel

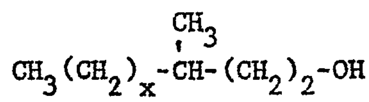

in der
x die vorstehend genannte Bedeutung hat oxidiert.

3. Verwendung der Verbindungen nach Anspruch 1 als Riechstoffe.

**Claims**

1. 3-methyl-aldehydes of the general formula :

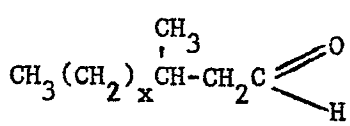

in which
x represents 6 or 7.

2. A process for the preparation of compounds of the general formula :

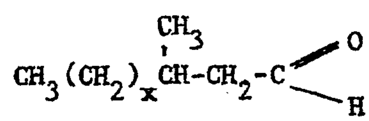

in which
x represents 6 or 7,
characterised in that
a) a carboxylic acid of the general formula :

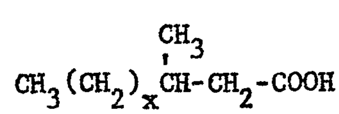

in which x has the afore-mentioned meaning, is reduced or

b) an alcohol of the general formula :

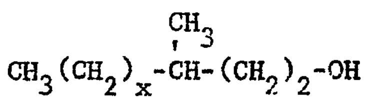

in which x has the afore-mentioned meaning, is oxidised.

3. The use of the compounds according to claim 1 as odorants.

**Revendications**

1. 3-méthylaldéhydes de formule générale

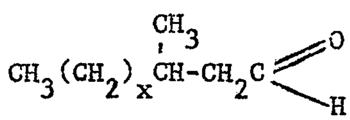

dans laquelle x est égal à 6 ou 7.

2. Procédé de préparation des composés de formule générale

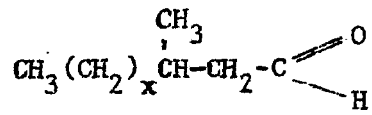

dans laquelle x est égal à 6 ou 7, caractérisé en ce que

a) on réduit un acide carboxylique de formule générale

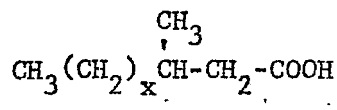

dans laquelle x a la signification indiquée ci-dessus, ou bien

b) on oxyde un alcool de formule générale

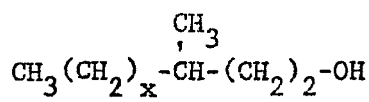

dans laquelle x a la signification indiquée ci-dessus.

3. Utilisation des composés selon la revendication 1 en tant que matières aromatiques.